(19) Europäisches Patentamt
European Patent Office
Office européen des brevets

(11) Numéro de publication : **0 644 241 A1**

(12) # DEMANDE DE BREVET EUROPEEN

(21) Numéro de dépôt : **94420249.8**

(22) Date de dépôt : **19.09.94**

(51) Int. Cl.⁶ : **C09C 1/40**, C09C 3/08, C09B 67/00, A61K 7/16, C11D 3/12, C08K 9/04, C04B 14/30, C01F 7/74

(30) Priorité : **21.09.93 FR 9311472**

(43) Date de publication de la demande : **22.03.95 Bulletin 95/12**

(84) Etats contractants désignés : **BE DE ES FR GB GR IE IT NL**

(71) Demandeur : **ALUMINIUM PECHINEY
Immeuble Balzac
10, place des Vosges
La Défense 5
F-92400 Courbevoie (FR)**

(72) Inventeur : **Alary, Jean-André
20, avenue Jean Moulin
F-13120 Gardanne (FR)**
Inventeur : **Soirat, Arnaud
Villa Marjolaine,
36, avenue Paul Cézanne
F-13090 Aix en Provence (FR)**

(74) Mandataire : **Mougeot, Jean-Claude et al
PECHINEY
28, rue de Bonnel
F-69433 Lyon Cedex 03 (FR)**

(54) **Hydrate d'alumine contenant un additif colorant, procédé de production et applications.**

(57) Hydrate d'alumine à l'état cristallisé ou amorphe ou en solution dans un solvant approprié tel qu'une liqueur acide ou alcaline caractérisé en ce qu'il contient au moins un additif colorant, solide ou solubilisé ou pouvant se disperser dans un solvant approprié tel qu'une solution acide, neutre ou alcaline, susceptible de modifier dans toute la plage de longueurs d'onde du spectre visible la coloration de l'hydrate d'alumine en vue d'améliorer de façon durable ses propriétés de blancheur lors de ses applications ultérieures.

L'additif colorant, dont la teneur pondérale dans l'hydrate est d'au moins 5 ppm et de préférence de 10 à 60 ppm, peut être un pigment comme la phtalocyanine de cuivre et est choisi en fonction de la coloration à corriger mais aussi de sa stabilité dans le milieu réactionnel où est mis en oeuvre l'hydrate d'alumine.

EP 0 644 241 A1

## DOMAINE TECHNIQUE

La présente invention concerne les hydrates d'alumine, notamment le trihydrate et le monohydrate d'alumine, à l'état cristallisé ou amorphe ou en solution dans un solvant approprié tel qu'une liqueur acide ou alcaline, contenant au moins un additif colorant destiné à adapter les caractéristiques de blancheur de l'hydrate en fonction des caractéristiques de blancheur ou de coloration du produit fini dans la composition duquel il intervient.

## ETAT DE LA TECHNIQUE

Hormis son emploi bien connu à l'état calciné, comme alumine métallurgique pour la production d'aluminium par electrolyse ignée, l'hydrate d'alumine sous forme de trihydrate (appelé aussi hydrargillite ou trihydroxyde d'aluminium) ou sous forme partiellement deshydratée, a trouvé par ses propriétés intrinsèques d'autres applications dans des domaines très variés.

Ainsi, par son caractère amphotère le trihydrate d'alumine, soluble donc en milieu acide et basique, s'avère un produit intermédiaire particulièrement bien adapté pour la fabrication des sels cristallisés d'aluminium notamment des sulfates et chlorures d'aluminium ou des aluminates alcalins utilisés dans le traitement des eaux comme agents d'absorption et de coagulation des impuretés minérales: phosphates, fluorures, silicates alcalins ou alcalino-terreux ou bien encore dans le collage du papier comme agent hydrofuge.

En solution sous forme d'aluminate de sodium, le trihydrate d'alumine avec le silicate de sodium constitue la matière de base de la fabrication des zéolites très utilisées pour la purification des gaz et des solvants mais dont les propriétés d'échangeur d'ions ont trouvé un important débouché dans les lessives, en substitution au moins partielle aux polyphosphates de sodium, pour fixer les cations $Ca^{++}$ et $Mg^{++}$ et abaisser la dureté de l'eau.

A l'état cristallisé, dans la mesure où il présente la granulométrie requise, le trihydrate d'alumine trouve de nombreuses applications comme charge:

- charge abrasive dans les pâtes dentifrice où sa dureté inférieure à celle de l'émail lui permet d'attaquer la plaque dentaire sans altérer l'émail des dents.
- charge ignifuge dans les matières plastiques étant donné que la décomposition endothermique du trihydrate d'alumine intervient précisément dans la plage de combustion exothermique de nombreux plastiques et élastomères et que l'alumine de transition issue de cette décomposition absorbe les produits de combustion limitant le dégagement des particules et fumées toxiques.
- charge dans la fabrication des marbres artificiels par mélange notamment avec des résines polyester non saturées où son indice de réfraction très voisin de celui desdites résines permet de conserver un haut degré de transparence.

Dans toutes ces applications l'emploi d'hydrate d'alumine est justifié par au moins une propriété spécifique mais ce choix ne doit pas être remis en cause par des effets secondaires mal maîtrisés susceptibles par exemple d'altérer ou de modifier les propriétés du produit final dans la composition duquel intervient cet hydrate.

A cet égard un facteur très important à prendre en compte dans de nombreuses applications de 1' hydrate d'alumine, est sa blancheur mesurée généralement par un indice déterminé colorimétriquement par rapport à un produit de référence en l'occurence de l'oxyde de magnésium ou du sulfate de baryum de très haute pureté. De fait cette blancheur doit être suffisante pour ne pas modifier l'aspect du produit final notamment pour tous les exemples d'application précédemment énumérés.

Ainsi les sels d'aluminium et en particulier le sulfate d'aluminium destiné au traitement de l'eau doivent présenter la plus grande blancheur possible.

Si la présence d'une très légère coloration provenant du trihydrate utilisé comme charge dans les pâtes dentifrices ou les matériaux ignifuges n'est pas absolument redhibitoire, il n'en est pas de même dans la fabrication des marbres synthétiques ou des zéolites à blancheur contrôlée entrant notamment dans la composition des lessives.

Ainsi le brevet EP 0279575 relatif à la fabrication de marbre artificiel par mélange de trihydrate d'alumine et de résine polyester non saturé considère qu'un trihydrate dont l'indice de blancheur est inférieur à 90 (l'indice de référence 100 étant attribué à l'oxyde de magnésium pur) ne peut être utilisé comme charge dans la fabrication des marbres synthétiques parce qu'il confère au produit final une légère teinte jaunâtre ou rougeâtre.

De même dans la fabrication des zéolites toute coloration parasite apportée au produit final par les constituants et notamment le trihydrate d'alumine est exclue. Pour qualifier le degré de blancheur on utilise généralement le système espace couleur de HUNTER officialisé en 1964 par la commission internationale de l'éclairage. Ce système représente dans l'espace selon 3 directions perpendiculaires 3 paramètres qui mesurent respectivement:

- la variation de blancheur (ou à l'inverse d'opacité) par un indice L positif ou négatif dont la valeur doit être supérieure à 97 pour les zéolites de lessive par référence à une valeur maximale de 100
- la variation de chromaticité mesurée sur les 2 autres axes de coordonnées respectivement par les coefficients a et b positifs ou négatifs, où a représente le degré de coloration moyen entre le rouge et sa couleur complémentaire verte et doit être dans le cas des zéolites pour lessive compris entre - 0,5 et + 0,5 et où b représente le degré de coloration moyen entre le bleu et sa couleur complémentaire jaune et doit être dans ce cas compris entre - 0,5 et 1.

En fait il est maintenant bien connu que ces irrégularités de blancheur du produit sont dues en partie à la présence d'impuretés qui absorbent sélectivement certaines longueurs d'onde du spectre de lumière blanche incidente de sorte que le faisceau émergeant de lumière est modifié et prend la couleur complémentaire de la couleur sélectivement absorbée. Ce phénomène d'absorption est particulièrement fréquent pour les longueurs d'onde situées en limite du spectre visible. Ainsi l'absorption de lumière bleue entre 4800 ± 300 A provoque l'apparition de la couleur complémentaire jaune alors qu'à l'autre extrémité du spectre visible l'absorption de lumière rouge à 6600 ± 300 A provoque un verdissement du produit.

Dans le cas du trihydrate d'alumine obtenu par attaque sodique de la bauxite selon le procédé Bayer puis décomposition en présence d'amorce de la liqueur sursaturée d'aluminate de sodium issue de l'attaque, les taux d'impuretés organiques ou minérales peuvent varier significativement en fonction de l'origine et de la composition initiale des bauxites ainsi que des conditions spécifiques du traitement. Ces fluctuations rendent donc très problématiques l'emploi d'un tel trihydrate d'alumine pour des applications où les critères d'aspect et notamment de blancheur sont déterminants.

Pour remédier à ces difficultés l'homme du métier est relativement démuni, car la solution consistant à préparer un trihydrate d'alumine de haute pureté est très onéreuse à l'échelle industrielle et cela quel que soit le procédé utilisé: redissolution alcaline d'une poudre d'aluminium très pure ou purification du trihydrate d'alumine issu du procédé Bayer par remise en solution et élimination des impuretés par précipitation sélective ou absorption sur résine échangeuse d'ions. A noter également que des essais de lavage intensif du trihydrate en vue d'entraîner certaines impuretés dont les matières organiques, se sont avérés inefficaces pour modifier les caractéristiques de blancheur du trihydrate. Cela a conduit à sélectionner selon les applications les lots de trihydrate d'alumine en fonction de la nature et de la teneur des impuretés résiduelles, c'est-à-dire en fait, en fonction des lots de bauxite utilisée comme matière première. Cette sélection constitue évidemment une contrainte d'exploitation très lourde.

Quant à la méthode consistant à corriger le défaut de blancheur du trihydrate par un pigment elle est exclue par l'enseignement très explicite du brevet EP 0279575 (page 2 lignes 29 à 32) indiquant que l'ajout d'un pigment décolorant au trihydrate destiné à être mélangé avec la résine est inopérant pour restituer au produit final la blancheur ou la coloration initialement désirée. Ce document confirme par ailleurs (page 2 lignes 33 à 35 et page 5 lignes 11 à 13) que le trihydrate d'alumine avant usage doit être débarrassé des matières organiques contenues par des moyens variés adaptés, mais sans préciser lesquels.

## PROBLEME POSE

En conclusion l'obtention dans des conditions industrielles économiquement viables d'hydrate d'alumine qui satisfasse aux critères de blancheur exigés par le produit final dans la composition duquel il rentre, demeure un problème essentiel à résoudre pour l'homme du métier.

## OBJET DE L'INVENTION

La présente invention apporte une solution en préconisant l'emploi d'hydrate d'alumine industriel, issu par exemple du procédé Bayer et donc d'un coût de production relativement bas, dont les caractéristiques de blancheur sont corrigées de façon durable par au moins un additif colorant en fonction des caractéristiques de blancheur ou de coloration désirées pour le produit final dans la composition duquel il rentre. Par les termes "corrigées de façon durable" il faut comprendre que les caractéristiques obtenues après fixation de l'additif colorant sont conservées dans le temps du fait que ledit additif présente une bonne résistance au vieillissement accéléré occasionné par les agents extérieurs à l'action desquels il est soumis, comme les radiations solaires ou les variations importantes et brutales de température.

Plus précisément l'invention concerne un hydrate d'alumine à l'état cristallisé ou amorphe ou en solution dans un solvant approprié tel qu'une liqueur acide ou alcaline, caractérisé en ce que l'hydrate d'alumine contient au moins un additif colorant, solide ou solubilisé ou pouvant se disperser dans un solvant approprié tel qu'une solution acide, neutre ou alcaline, susceptible de modifier dans toute la plage de longueurs d'onde du spectre visible la coloration de l'hydrate d'alumine en vue d'améliorer de façon durable ses propriétés de

blancheur lors de ses applications ultérieures.

L'invention a aussi pour objet un procédé d'élaboration de l'hydrate d'alumine avec son additif introduit en quantité prédéterminée pour avoir la meilleure efficacité possible dans le produit final, qui peut être notamment un marbre artificiel, une zéolite, du sulfate d'aluminium, voire une matière plastique ignifugée ou une pâte dentifrice.

En effet l'invention repose sur le triple constat suivant:

a) Il est possible de modifier et donc d'améliorer les caractéristiques de blancheur d'un produit par la correction des caractéristiques de blancheur d'un des constituants intermédiaires de base qu'est l'hydrate d'alumine, dans la mesure où toutes les autres caractéristiques (degré de pureté, granulométrie, texture) de l'hydrate et des autres constituants du produit fini sont stabilisées.

b) Cette correction par un additif colorant, qui dans le cas présent de l'hydrate d'alumine peut se réaliser sur le produit à l'état cristallisé ou amorphe ou en solution dans un solvant approprié telle qu'une liqueur acide ou alcaline, est précise et durable au niveau du produit fini si les propriétés optiques adéquates sont évidemment obtenues après élaboration de ce produit fini. Ceci implique le choix d'un additif dont les propriétés optiques ne soient pas trop dégradées dans les conditions d'élaboration du produit final (PH, température), ou dont les produits de dégradation ont un impact positif sur la blancheur du produit final, si l'on veut procéder aux ajustements quantitatifs nécessaires sans gros risque d'erreur.

c) Cette correction est sélective, c'est-à-dire qu'elle peut s'appliquer à un paramètre précis de chromaticité tel que défini dans le système de couleur de HUNTER où les coefficients a et b peuvent être corrigés pratiquement indépendamment l'un de l'autre pour réduire sinon annihiler l'effet d'une impureté sur l'un d'eux, et cela sans fortement diminuer le coefficient de blancheur L.

Ainsi l'additif colorant, quand il n'est pas soluble dans la liqueur contenant le trihydrate d'alumine en suspension ou en solution, doit pouvoir se disperser aisément dans cette liqueur afin de former une fine suspension homogène assurant un recouvrement uniforme des particules d'hydrate en suspension ou qui seront ultérieurement précipitées à partir de ladite liqueur.

Cet additif colorant, qui peut être un pigment organique ou minéral, est contenu dans la proportion pondérale d'au moins 5 ppm rapportée à l'hydrate d'alumine à l'état solide ou en solution. Cette proportion pondérale est de préférence comprise entre 10 et 60 ppm. Cet additif colorant peut être choisi dans le groupe des phtalocyanines comme la phtalocyanine de cuivre $C_{32}$ $H_{16}$ Cu $N_8$ (CI nom générique Pigment blue 15:3) quand l'on désire compenser l'absorption de la couleur bleue dans la plage de longueur d'onde $4800 \pm 500$ A. Toutefois en milieu acide concentré notamment en milieu sulfurique on utilisera avantageusement un additif colorant plus stable choisi par exemple dans le groupe des dioxazines comme le violet de dioxazine.

A l'autre extrémité du spectre visible, si l'on désire compenser l'absorption de la couleur rouge dans la plage de longueur d'onde $6600 \pm 500$ A on utilisera par exemple des dérivés monoazoïques aromatiques et plus spécialement le sel de manganèse de l'acide 4 [(5 chloro - 4 -méthyl 2 sulfophenyl) azo] - 3 - hydroxy - 2 - naphtalène carboxylique de formule $C_{18}$ $H_{13}$ Cl $N_2$ $O_6$ S. Mn (CI nom générique pigment red 48:3).

Ainsi la coloration légèrement jaunâtre de certains lots de trihydrate d'alumine issus de bauxites à forte teneur en matières organiques, jugés impropres à l'utilisation comme charge dans les marbres artificiels, a pu être éliminée par lavage final à l'eau ou avec une solution sodique diluée (10 à 20 g de $Na_2O$ par litre) contenant de la phtalocyanine de cuivre recyclée jusqu'à fixation de la totalité de ce colorant bleuté sur les grains de trihydrate. Dans le cas présent ce taux de fixation correspondait à un apport pondéral d'environ 10 ppm de colorant rapporté à Al $(OH)_3$ et était contrôlé colorimétriquement par la disparition progressive de la coloration des eaux de lavage en cours de recyclage par comparaison à une eau de lavage exempte d'additif colorant. Par ailleurs l'indice de blancheur L du trihydrate d'alumine supérieur à 90 était conservé autorisant l'emploi de ces lots initialement inutilisables comme charge de marbre synthétique.

De la même façon la coloration jaunâtre de lots de trihydrate d'alumine destinés à la fabrication de zéolite, liée cette fois à la présence de CaO résiduel (jusqu'à 500 ppm) provenant d'un ajout volontaire de chaux lors du traitement de certaines bauxites en vue notamment d'améliorer le rendement d'extraction de l'hydrate d'alumine, a pu être éliminée par fixation sur le trihydrate d'alumine de plus de 100 ppm d'additif colorant à base de phtalocyanine de cuivre, de sorte que dans le produit final le coefficient de chromaticité b = 2,20 mesure de ce jaunissement a pu être ramené au voisinage de 0 sans que les autres paramètres L et a, par ailleurs corrects, aient été significativement modifiés.

Enfin dans le domaine de la fabrication des sels cristallisés d'aluminium et notamment du sulfate d'aluminium destiné au traitement des eaux, il a été possible d'améliorer la blancheur du sulfate d'aluminium issu de la cristallisation d'une solution saturée d'hydrate d'alumine dans $H_2$ $SO_4$ concentré en introduisant dans ladite solution saturée de 10 à 20 ppm d'additif à base du sel organique de manganèse $C_{18}$ $H_{13}$ Cl $N_2$ $O_6$ S - Mn.

En effet la légère tendance au verdissement du sulfate d'aluminium issu de certains lots de trihydrate d'alumine dont le coefficient de chromaticité a trop négatif a pu être ainsi corrigée sans forte dégradation des autres

paramètres L et b.

A l'inverse, une légère tendance au jaunissement du sulfate d'aluminium cristallisé, préparé dans les mêmes conditions mais à partir d'un lot de trihydrate d'alumine plus chargé en impuretés favorisant l'augmentation du coefficient b au-delà de 1, a pu être corrigée en introduisant dans la solution saturée d'alumine dans $H_2SO_4$ concentré, de 15 à 40 ppm de violet de dioxazine (CI nom générique pigment violet 23). Ainsi, le coefficient b de 1,62 a pu être réglé entre 0,00 et 0,96 alors que les autres coefficients L et a n'étaient pas modifiés de façon significative en restant respectivement compris entre 97,2 et 97,7 pour L et entre -0,1 et -0,2 pour a, c'est-à-dire tout à fait similaires aux coefficients caractéristiques du produit de référence.

Il ressort de ces différents essais que la correction de blancheur de l'hydrate d'alumine à l'état cristallisé, amorphe ou en solution dans un solvant approprié telle qu'une solution aqueuse acide ou alcaline est tout aussi efficace et peut donc intervenir indifféremment à divers stades d'élaboration du produit dès l'instant que l'additif colorant reste suffisamment stable dans le milieu réactionnel, ou que ses produits de dégradation ont un impact positif sur la blancheur du produit final.

## MISE EN OEUVRE DE L'INVENTION

L'invention sera mieux comprise par la description détaillée des conditions d'introduction de l'additif colorant dans l'hydrate d'alumine le plus fréquemment utilisé pour les applications qui requièrent des caractéristiques de blancheur ou de coloration précises, à savoir le trihydrate d'alumine tant à l'état solide qu'en solution dans un solvant approprié tel qu'une liqueur alcaline ou acide, ledit trihydrate étant généralement issu du traitement des bauxites selon le procédé Bayer qui est le plus connu des procédés industriels de fabrication d'hydrates d'alumine et d'alumine.

La matière première utilisée est le minerai de bauxite qui contient de 40 à 60% en poids d'alumine ($Al_2O_3$) à l'état d'hydrate d'alumine hydrargillite, boehmite, diaspore, mais également des impuretés dont la nature et la teneur varient selon la provenance des minerais. On trouve néanmoins régulièrement les éléments métalliques suivants fer, calcium, silicium, titane, voire vanadium et gallium, présents à l'état d'oxyde mais également des matières organiques dont l'élimination plus ou moins complète lors du cycle de traitement Bayer conditionne le type d'application ultérieure du trihydrate d'alumine et de l'alumine.

Le minerai de bauxite concassé puis finement broyé est attaqué par une solution sodique fortement concentrée (140 à 200 g $Na_2O$/litre) contenant un peu d'alumine en solution, s'agissant d'une liqueur recyclée appauvrie en alumine après précipitation du trihydrate lors d'une étape précédente.

La dissolution complète de l'alumine à l'état d'hydrate est obtenue à une température supérieure à 200°C et généralement comprise entre 235°C et 250°C selon la réaction

$$Al(OH)_3 + NaOH \rightleftharpoons Na\,Al(OH)_4$$

La solubilisation de l'alumine présente à l'état de monohydrate sous forme de boehmite ou de diaspore est obtenue par la forte concentration en soude et la température élevée.

Après séparation par décantation et filtration de la liqueur sursaturée en alumine solubilisée à l'état d'aluminate de sodium, des résidus solides riches en oxydes de fer ou boues rouges, cette liqueur sursaturée est refroidie en présence d'amorce de trihydrate d'alumine pour favoriser sa décomposition par précipitation entre 80°C et 50°C de la majeure partie du trihydrate solubilisé. Les constituants de la suspension en fin de décomposition trihydrate d'alumine et liqueur décomposée, c'est-à-dire appauvrie en alumine solubilisée, sont séparés par filtration. La liqueur décomposée est recyclée comme liqueur d'attaque de la bauxite alors qu'une fraction très importante (9/10) du trihydrate est recyclée comme amorce de décomposition et que l'autre fraction de granulométrie plus grossière, représentant la production est lavée sur filtre et éventuellement séchée, avant d'être mise en contact avec l'additif colorant.

A ce stade les principales impuretés contenues dans le trihydrate sont :

- La soude de l'ordre de 1500 ppm de $Na_2O$ dont la plus grande partie est prisonnière du réseau cristallin.
- La silice de l'ordre de 100 ppm généralement combinée à l'alumine sous forme de silicoaluminate de sodium.
- La chaux à des teneurs pouvant varier de quelques dizaines de ppm à plusieurs centaines de ppm quand elle a volontairement été introduite, au cours de l'attaque de la bauxite pour améliorer le rendement de solubilisation de l'alumine, ou après attaque pour améliorer la séparation de la liqueur des résidus insolubles à la décantation ou à la filtration.
- Le fer sous forme de $Fe_2O_3$ a des teneurs généralement comprises entre 100 et 200 ppm.

- Les matières organiques, dont des matières humiques plus ou moins dégradées, qui se dissolvent dans la liqueur sodique d'attaque et précipitent ensuite dans le trihydrate d'alumine. Leur teneur peut varier dans le trihydrate d'alumine de moins de 100 ppm à plusieurs centaines de ppm selon la bauxite d'origine.

A noter que le taux de matières organiques du trihydrate n'est pas modifié significativement par les conditions de lavage.

Pour corriger les effets néfastes de ces impuretés et plus particulièrement de $Fe_2O_3$, de CaO et des matières organiques qui, présentes simultanément ou isolément en trop fortes quantités (300 à 400 ppm), provoquent une coloration généralement jaunâtre du trihydrate d'alumine ou des produits dans la composition duquel il rentre, on introduit, selon l'invention, une quantité déterminée d'additif colorant dans le trihydrate d'alumine cristallisé en procédant de la façon suivante:

Après filtration et lavage de la fraction de trihydrate d'alumine provenant de la décomposition d'une liqueur sursaturée d'aluminate de sodium en présence d'amorce et destiné à la production, on prélève une série d'échantillons représentatifs du lot de trihydrate d'alumine à traiter de poids P, pour détermination de la teneur pondérale optimale x en additif colorant, exprimée en ppm rapporté à $Al(OH)_3$, qu'il convient d'introduire dans le trihydrate à l'état solide ou en solution dans un solvant approprié tel qu'une liqueur acide ou alcaline afin d'obtenir dans le produit final fini les caractéristiques de blancheur optimales mesurées selon le système de couleur de Hunter.

Cette teneur pondérale x est déterminée empiriquement selon un mode opératoire propre à chaque produit fini et qui implique de connaître les conditions générales d'élaboration de ces produits.

Ce mode opératoire sera exolicité dans le cas, le plus intéressant pour l'invention, de la fabrication des zéolites A à blancheur contrôlée.

On détermine donc le poids p d'additif colorant à mettre en oeuvre pour traiter le lot de trihydrate d'alumine de poids P à l'état sec de telle sorte que

$$x\text{ppm} = 10^6 \frac{p}{P}$$

Ce poids p d'additif colorant est mis alors en contact avec le trihydrate d'alumine. Il est généralement introduit dans une solution aqueuse faiblement alcaline (10 à 23 g $Na_2O$/litre) utilisée comme eau de lavage et d'imprégnation du lot de trihydrate humide en attente de traitement. La solution est recyclée à travers la couche de trihydrate humide jusqu'à disparition de sa coloration (bleutée si l'additif est la phtalocyanine de cuivre) correspondant à la fixation quasi complète dudit additif colorant sur les grains de trihydrate. Généralement deux cycles suffisent pour capter la totalité de l'additif colorant.

Le contrôle de coloration est réalisé colorimétriquement par comparaison à une solution témoin exempte de colorant. Une fois l'additif colorant fixé à la surface des grains le trihydrate d'alumine est essoré et le cas échéant séché.

Une première variante du procédé, applicable quand l'additif colorant de poids p peut être solubilisé ou mis en suspension colloïdale dans un solvant approprié, sans effet nocif sur le trihydrate d'alumine précipité, consiste à pulvériser ledit solvant contenant l'additif sur le lot de trihydrate pendant le lavage sur filtre ou après filtration.

Une deuxième variante du procédé, applicable quand le trihydrate d'alumine séché doit subir une opération ultérieure de broyage ou de mélange avec d'autres composés solides, consiste à introduire l'additif colorant de poids p sous forme solide, solubilisé ou en suspension dans un solvant approprié, dans le broyeur ou le mélangeur contenant le lot de trihydrate sec, seul ou en mélange avec d'autres composés.

Une troisième variante du procédé, applicable quand le lot de trihydrate d'alumine avec son additif n'est pas utilisé directement à l'état solide mais doit être mis en solution, par exemple, dans une liqueur concentrée acide (solution sulfurique pour préparation de sulfate d'aluminium) ou basique (solution sodique pour préparation de zéolite), consiste à introduire directement l'additif colorant, sous forme solide, solubilisé ou en suspension dans un solvant approprié, dans le solvant contenant le trihydrate.

Ainsi, dans le cas des zéolites, silicoaluminates alcalins cristallisés en structures stables de formule générale :

$$M_{2/n}\,O.Al_2O_3.y\,SiO_2\,wH_2O$$

avec

n = valence du cation M

$y \geqq 2$

w : nombre de molécules d'eau de cristallisation

la correction de blancheur par un additif colorant apporté par le constituant de base qu'est le trihydrate d'alumine à l'état solide ou déjà en solution dans la soude est possible quelle que soit la structure de la zéolite dès

l'instant qu'elle doit dans son application satisfaire à des critères de blancheur. C'est plus précisément dans la fabrication de zéolite A pour lessive que l'invention trouve sa meilleure application.

**APPLICATION AUX ZEOLITES A**

Les zéolites A de formule chimique $Al_2O_3$. $Na_2O$. $2SiO_2$. n $H_2O$ (selon les conditions de séchage $1 \leqq n \leqq 8$ et généralement $4 \leqq n \leqq 5$) s'avèrent particulièrement efficaces comme échangeurs de cations et leur capacité d'échange très élevée notamment vis à vis du calcium (supérieure à 112 mg par g. de zéolite) et à un degré moindre du magnésium, les font utiliser dans les lessives dans la mesure où elles présentent par ailleurs une granulométrie et des caractéristiques de blancheur compatibles avec les exigences des fabricants de lessive. Ainsi il est essentiel que la répartition granulométrique soit resserrée autour d'un diamètre médian de 2 à 5 micromètres, suffisamment petit pour éviter les rétentions du produit dans les fibres du textile, mais suffisamment grand pour permettre une séparation aisée solide-liquide lors de la fabrication de la zéolite elle-même. En ce qui concerne la blancheur, la zéolite A doit être conforme aux spécifications des lessiviers qui utilisent généralement le système de couleur L, a, b de Hunter où L doit être supérieure à 97 et les 2 coefficients a et b de chromaticité définissant la couleur doivent être compris respectivement entre - 0,5 et + 0,5 et -0,5 et + 1.

En vue de déterminer la teneur optimale x en additif colorant du trihydrate d'alumine issu du cycle Bayer et destiné à la fabrication de zéolite A après dissolution en milieu sodique concentré et mélange avec une solution de silicate de sodium, une série d'échantillons représentatifs d'un lot de trihydrate d'alumine AH950 (dont les caractéristiques sont indiquées dans le tableau 1 ci-après) ont été additionnés d'additif colorant en teneurs pondérales croissantes de 0 à 60 ppm, puis transformés en autant d'échantillons de zéolite A qui ont été soumis aux tests de blancheur précédemment indiqués dans les conditions suivantes:

Tableau 1 -

| Caractéristiques AH 950 | |
|---|---|
| - $Al_2O_3$ $3H_2O$: | 98,7% sur sec soit $Al_2O_3$: 64,5% |
| - $Na_2O$ : | 920 ppm sur sec soit $Ga_2O_3$: 90 ppm |
| - $SiO_2$ : | 30 ppm sur sec soit ZnO : 90 ppm |
| - $Fe_2O_3$ : | 165 ppm sur sec |
| - CaO : | 105 ppm sur sec |
| - C oxalique: | 40 ppm sur sec |

A partir d'un échantillon primaire représentatif d'un lot de trihydrate AH950 conservé humide après lavage et essorage, en attente d'addition de colorant, on a constitué 7 échantillons identiques qui à l'exception de l'échantillon n° 1 repéré AHI et gardé comme témoin ont été additionnés de phtalocyanine de cuivre.

Les 4 échantillons AH2 - AH3 - AH4 et AH5 ont été respectivement additionnés de 10, 20, 25 et 60 ppm de phtalocyanine de cuivre préalablement introduite dans de l'eau ou dans une liqueur sodique diluée (20 g $Na_2O$/litre) suivant les 4 concentrations croissantes et cyclée jusqu'à sa fixation complète sur chaque échantillon contrôlée colorimétriquement par la disparition de la coloration bleutée de la liqueur recyclée. Les échantillons AH2 à AH5 ont été ensuite essorés.

Tous les échantillons ont été ensuite dissous dans une liqueur sodique concentrée pour former une solution d'aluminate de sodium prête pour la fabrication de zéolite.

Ce n'est qu'après dissolution dans la liqueur sodique concentrée que les 2 échantillons AH6 et AH7 ont été additionnés de phtalocyanine de cuivre à raison respectivement de 7 à 20 ppm rapportées à Al $(OH)_3$ dissous.

A partir des 7 échantillons de trihydrate d'alumine dissous dans la liqueur sodique concentrée on a préparé 7 échantillons de zéolite A repérés successivement Z1 à Z7 (Z1 correspondant à l'hydrate témoin AH1 et Z6, Z7 corresoondant aux hydrates AH6 et AH7 additionnés de phtalocyanine après dissolution en milieu sodique).

Compte tenu des contraintes de granulométrie (D 50% compris entre 2 et 5 micromètres et étalement mesuré par D 10% et D 90%), de pureté et d'homogénéité de structure, car il convient d'éviter la coprécipitation de produits secondaires parasites tels que les zéolites X ou Y, lorsque l'on utilise des solutions d'aluminate de sodium et de silicate de sodium trop diluées, ou que la sodalite, si l'on effectue la cristallisation à plus de

100°C en présence de soude contenant des ions chlorures et à une concentration en $Na_2O$ supérieure à 120 g/l.

Pour cela les conditions opératoires suivantes ont été choisies pour précipiter sélectivement la zéolite A selon la réaction.

$Na_2O$. $2SiO_2$ + x ($Al_2O_3$. y $Na_2O$) + z H2O ------> $Al_2O_3$. $Na_2O$. $2SiO_2$ . $nH_2O$ (zéolite A) + (x - 1) ($Al_2O_3$. y $Na_2O$) + (z - n+1)) $H_2O$ + 2 NaOH.

On réalise une composition liquide contenant:

- d'une part le trihydrate d'alumine additionné de l'additif colorant dissous dans la liqueur sodique concentrée sous forme d'aluminate de sodium dont le rapport Rp des concentrations $Al_2O_3$g/litre/$Na_2O$ caustique g/litre est compris entre 0,7 et 1,6 avec une concentration en $Al_2O_3$ comprise entre 60 et 450 g/l,
- d'autre part du silicate de sodium en solution dont le rapport Rp' des concentrations $SiO_2$g/l/$Na_2O$ g/l est compris entre 2 et 3,5 et de préférence compris entre 3 et 3,5 (car au voisinage de 2 on constate déjà une diminution du pouvoir séquestrant de la zéolite), alors que la concentration en $SiO_2$ doit être comprise entre 120 et 350 g/l et de préférence entre 130 et 170 g/l,

mélangés dans des proportions telles que le rapport Rp" des concentrations $Al_2O_3$ g/l/$SiO_2$ g/l soit supérieur à 1 et de préférence compris entre 1,1 et 1,9 pour qu'en fin de réaction les eaux mères soient uniquement constituées d'aluminate de sodium.

Les temps de précipitation puis de "mûrissement" dépendent de la concentration des 2 solutions de départ et de la température de précipitation. A une température comprise entre 80° et 100°C on précipite en quelques minutes un gel qui après homogénéisation et maintien en suspension cristallise au bout de 2 à 16 heures. A noter qu'à moins de 70°C la zéolite A ne précipite pas sans l'aide d'amorce. Après filtration et lavage à l'eau chaude les cristaux de silicoaluminate de sodium sont sèchés à moins de 120°C pour ne pas déshydrater la zéolite A et généralement entre 80 et 100°C pendant 8 à 12 heures oour lui conserver a à 5 molécules d'eau de cristallisation.

C'est ainsi que 100 ml de chacun des 7 échantillons (AH1 à AH7), dissous dans la liqueur concentrée sodique de composition moyenne,

| | |
|---|---|
| $Al_2O_3$ | = 200 g/l |
| $Na_2O$ caustique | = 180 g/l |
| Rp | = 1,11 |

ont été mis en contact sous agitation mécanique vigoureuse (650 t/m) dans un réacteur à double enveloppe thermostatée par de l'eau à 82°C avec 110 ml d'une solution de silicate de sodium de composition moyenne

| | |
|---|---|
| $Na_2O$ caustique | = 50 g/l |
| $SiO_2$ | = 165 g/l |
| Rp' | = 3,3 |

Lors du mélange le rapport pondéral Rp" des concentrations $Al_2O_3$ g/l/$SiO_2$ g/l était de 200x0,1/165x0,11 soit Rp" = 1,1

Après 10 minutes d'agitation intense pour homogénéiser le gel formé on a laissé cristalliser ce gel pendant 2,5 heures à 80°C sous une agitation suffisante pour maintenir les cristaux en suspension. Par filtration sur buchner avec verre fritté de porosité 4, suivie d'un lavage des cristaux de silicoaluminate par 100 ml d'eau permutée chaude on a recueilli 190 ml d'une liqueur de filtration (eau-mère + eau de lavage) de composition moyenne :

| | |
|---|---|
| $Al_2O_3$ | = 20 g/l |
| $Na_2O$ | = 60 g/l |
| $SiO_2$ | = néant |

On a constaté d'une part que la liqueur mère ne comportait pas de silice solubilisée donc que toute la silice était combinée dans la zéolite, d'autre part que la liqueur mère ne comportait pas de trace d'additif colorant, donc que celui-ci était intégralement fixé sur la zéolite.

Après séchage des cristaux de silicoaluminate pendant 12 heures à 80°C les 7 échantillons de zéolite Z1 à Z7 d'un poids d'environ 55 g avaient la composition pondérale moyenne d'une zéolite A à savoir:

| | | | |
|---|---|---|---|
| $Al_2O_3$ | 29 à 30% | $Na_2O$ | 15 à 16% |
| $SiO_2$ | 34 à 35% | $H_2O$ | 19 à 20% |

Le spectre de rayons X sur les 7 échantillons a confirmé l'existence d'une structure de zéolite A exempte de zéolite X ou Y et de sodalite. Par ailleurs les résultats des mesures de blancheur et de coloration par spec-

trocolorimétrie à partir d'une source lumineuse avec $BaSO_4$ comme référence externe sont consignés dans le tableau 2 ci-après avec les mesurés de répartition granulométrique (D 50 et étalement) par granulométrie laser.

Tableau 2

| Réf. zéolite | Ajout colorant | Teneur ppm/Al(OH)3 | Blancheur - coloration | | | Granulométrie µm | | |
|---|---|---|---|---|---|---|---|---|
| | | | L | a | b | D50 | D10 | D90 |
| Z1 | néant | 0 | 98,4 | -0,03 | 1,31 | 3,77 | 1,18 | 9,19 |
| Z2 | AH2 cristallisé | 10 | 97,6 | -0,36 | 0,84 | 3,54 | 1,15 | 7,17 |
| Z3 | AH3 cristallisé | 20 | 97,8 | -0,40 | 0,52 | 3,95 | 1,22 | 8,87 |
| Z4 | AH4 cristallisé | 25 | 97,9 | -0,40 | 0,28 | 3,31 | 1,19 | 6,41 |
| Z5 | AH5 cristallisé | 60 | 96,5 | -0,92 | 1,03 | 3,61 | 1,18 | 7,02 |
| Z6 | AH6 solution | 7 | 97,8 | -0,30 | 0,92 | 3,89 | 1,21 | 9,61 |
| Z7 | AH7 solution | 20 | 97,9 | -0,40 | 0,51 | 3,99 | 1,21 | 8,53 |

Ces résultats appellent les commentaires suivants:

Les granulométries des 7 échantillons sont très voisines et confirment les similitudes de composition et de structure déterminées aux rayons X, en revanche on remarque des différences notables dans les caractéristiques de blancheur et de chromaticité. Ainsi sur les 4 zéolites Z2 à Z5 où l'addition de phtalocyanine de cuivre a été effectuée sur le trihydrate cristallisé AH2 à AH5 on observe une légère diminution du coefficient L de blancheur qui reste néanmoins tout à fait acceptable avec 97,8 pour des ajouts de 20 à 25 ppm de colorant. Il en est de même du coefficient a qui diminue faiblement et reste acceptable à -0,40 pour un ajout de 25 ppm de colorant alors que simultanément le coefficient b, qui en l'absence d'ajout dépasse nettement 1 traduisant une absorption excessive de la plage des longueurs d'onde située dans les bleus, est abaissé significativement entre 0,5 et 0,3, valeurs tout à fait acceptables que l'on n'a pu obtenir avec les trihydrates sans additif colorant même s'ils sont très purs.

Pour les zéolites Z6 à Z7 où l'addition de phtalocyanine de cuivre a été effectuée sur le trihydrate en solution sodique on n'observe pas à teneur identique (Z3 et Z7) de différences significatives dans l'évolution des caractéristiques de blancheur par rapport à celles constatées avec ajout de colorant au trihydrate cristallisé. On peut déduire qu'une teneur de x = 20 ppm de phtalocyanine de cuivre paraît la plus adaptée pour corriger le coefficient b sans modifier notablement les 2 autres coefficients a et L du lot de trihydrate AH950 en attente de traitement et que cette addition peut se faire avec la même efficacité sur le lot de trihydrate d'alumine cristallisé humide après lavage ou après essorage que sur le lot de trihydrate d'alumine déjà en solution sous forme d'aluminate de sodium dans la liqueur sodique concentréeprête à l'élaboration de la zéolite A.

Le procédé de fixation de l'additif colorant sur le trihydrate d'alumine qui vient d'être décrit avec ses différentes variantes s'applique évidemment aux hydrates d'alumine partiellement déshydratés et notamment au monohydrate d'alumine A100H.

De façon plus exhaustive, quelque soit le mode d'obtention de l'hydrate d'alumine à l'état cristallisé, amorphe ou en solution dans un solvant approprié qui peut être un solvant organique, l'homme du métier muni de

ces informations est en effet à même de choisir en fonction des conditions d'élaboration et d'utilisation du produit final (en particulier du milieu réactionnel) ainsi que des caractéristiques de blancheur à corriger, d'une part l'additif colorant adéquat, d'autre part le mode d'introduction dans l'hydrate le plus approprié.

Ainsi dans le cas de monohydrate d'alumine très finement cristallisé obtenu par exemple par autoclavation en présence de vapeur d'eau de trihydrate d'alumine ou par cristallisation d'un gel de boehmite résultant de l'acidification d'une liqueur d'aluminate de sodium la mise en contact avec l'additif colorant peut s'effectuer aisément par remise en solution ou en suspension de l'hydrate dans un solvant approprié contenant déjà l'additif colorant solubilisé ou en suspension ou en procédant dans l'ordre inverse, le solvant étant ensuite éliminé, après filtration éventuelle, par tout moyen de séchage connu tel que l'atomisation ou l'étuvage.

## AVANTAGE DECOULANT DE L'INVENTION

La possibilité, par simple ajout d'une quantité déterminée d'additif colorant à l'hydrate d'alumine industriel, d'améliorer sélectivement certaines caractéristiques de blancheur du produit final dans la composition duquel il rentre, constitue pour le fabricant d'hydrate d'alumine un avantage économique très important en terme de coût mais aussi de souplesse d'exploitation par rapport aux produits réalisés selon l'art antérieur.

Cela implique toutefois de bien connaître les conditions d'élaboration et d'utilisation du produit final pour choisir l'additif colorant approprié à la correction sélective recherchée qui, même après une éventuelle dégradation partielle lors de l'élaboration du produit final, conservera à celui-ci lors de son utilisation ultérieure les propriétés optiques adéquates.

## Revendications

**1)** Hydrate d'alumine à l'état cristallisé ou amorphe ou en solution dans un solvant approprié, tel qu'une liqueur acide ou alcaline, caractérisé en ce qu'il contient au moins un additif colorant, solide ou solubilisé ou pouvant se disperser dans un solvant approprié tel qu'une solution acide, neutre ou alcaline, susceptible de modifier dans toute la plage de longueurs d'onde du spectre visible la coloration de l'hydrate d'alumine en vue d'améliorer de façon durable ses propriétés de blancheur lors de ses applications ultérieures.

**2)** Hydrate d'alumine selon la revendication 1 caractérisé en ce que l'additif colorant est un pigment organique ou minéral.

**3)** Hydrate d'alumine selon la revendication 1 ou 2 caractérisé en ce qu'il contient une proportion pondérale d'au moins 5 ppm et de préférence 10 à 60 ppm d'additif colorant.

**4)** Hydrate d'alumine selon l'une quelconque des revendications 1 à 3 caractérisé en ce que l'additif colorant est choisi dans le groupe des phtalocyanines.

**5)** Hydrate d'alumine selon la revendication 4 caractérisé en ce que l'additif colorant est la phtalocyanine de cuivre $C_{32}\ H_{16}\ Cu\ N_8$.

**6)** Hydrate d'alumine selon l'une quelconque des revendications 1 à 3 caractérisé en ce que l'additif colorant est choisi dans le groupe des dérivés azoïques aromatiques.

**7)** Hydrate d'alumine selon la revendication 6 caractérisé en ce que l'additif colorant est le sel de manganèse de l'acide 4 - [(5 chloro - 4 - méthyl - 2 sulfophényl) azo] - 3 - hydroxy - 2 -naphtalene carboxylique de formule chimique: $C_{18}\ H_{13}\ ClN_2O_6\ S.\ Mn$.

**8)** Hydrate d'alumine selon l'une quelconque des revendications 1 à 3 caractérisé en ce que l'additif colorant est choisi dans le groupe des dioxazines comme le violet de dioxazine.

**9)** Composition liquide contenant:

- d'une part du trihydrate d'alumine, selon l'une quelconque des revendications 1 à 5, dissous dans une liqueur sodique concentrée sous forme d'aluminate de sodium dont le rapport Rp des concentrations $Al_2O_3$ g/l/ $Na_2O$ g/l est compris entre 0,7 et 1,6 avec une concentration en $Al_2O_3$ comprise entre 60 et 450 g/litre,
- d'autre part du silicate de sodium en solution dont le rapport Rp' des concentrations $SiO_2$ g/l/$Na_2O$ g/l est compris entre 2 et 3,5 et de préférence compris entre 3 et 3,5 avec une concentration en $SiO_2$ comprise entre 120 et 350 g/l et de préférence entre 130 et 170 g/l, mélangés en proportions telles que le rapport pondéral Rp" des concentrations $Al_2O_3$ g/l/$SiO_2$ g/l soit supérieur à 1 et de préférence compris entre 1,1 et 1,9.

**10)** Procédé de fabrication d'hydrate d'alumine contenant au moins un additif colorant selon l'une quelconque des revendications 1 à 8, formé essentiellement de trihydrate d'alumine provenant de la décomposition d'une liqueur sursaturée d'aluminate de sodium en présence d'amorce caractérisé en ce que, après filtration et lavage de la fraction de trihydrate destiné à la production, on prélève une série d'échantillons représentatifs

du lot de trihydrate à traiter de poids P pour détermination de la teneur optimale x en additif colorant, exprimée en ppm rapportées au poids P de $Al(OH)_3$, qu'il convient d'introduire dans le trihydrate à l'état solide ou en solution dans un solvant approprié tel qu'une liqueur acide ou alcaline afin d'obtenir dans le produit fini les caractéristiques de blancheur optimales mesurée selon le système de couleur de HUNTER, d'où l'on déduit le poids p d'additif colorant à mettre en contact avec le lot de trihydrate d'alumine.

**11)** Procédé selon revendication 10 caractérisé en ce que le poids p d'additif colorant est introduit dans une solution aqueuse faiblement alcaline utilisée comme eau de lavage et d'imprégnation du lot de trihydrate de poids P, que l'on recycle à travers la couche de trihydrate humide jusqu'à disparition de sa coloration correspondant à la fixation quasi complète dudit colorant sur les grains de trihydrate, le contrôle de coloration étant réalisé colorimétriquement par comparaison à une solution témoin exempte de colorant.

**12)** Procédé de fabrication d'hydrate d'alumine selon revendication 10 caractérisé en ce que le poids p d'additif colorant est solubilisé ou mis en suspension colloïdale dans un solvant approprié qui est pulvérisé sur le trihydrate d'alumine pendant le lavage sur filtre ou après filtration.

**13)** Procédé de fabrication d'hydrate d'alumine selon revendication 10 caractérisé en ce que le poids p d'additif colorant, sous forme solide, solubilisé ou en suspension dans un solvant approprié est introduit dans un broyeur ou un mélangeur contenant le lot de trihydrate sec, seul ou en mélange avec d'autres composés solides.

**14)** Procédé de fabrication d'hydrate d'alumine selon revendication 10 caractérisé en ce que le poids p d'additif colorant, sous forme solide, solubilisé ou en suspension dans un solvant approprié, est introduit directement dans le solvant tel qu'une liqueur concentrée acide ou basique contenant le lot de trihydrate d'alumine en solution.

**15)** Procédé de fabrication d'hydrate d'alumine contenant au moins un additif colorant selon l'une quelconque des revendications 1 à 8 caractérisé en ce que la mise en contact avec l'additif colorant est effectuée par remise en solution ou en suspension de l'hydrate dans un solvant approprié contenant déjà l'additif colorant en solution ou en suspension, ou en procédant dans l'ordre inverse, le solvant étant ensuite éliminé, après filtration éventuelle, par tout moyen de séchage connu.

**16)** Application d'hydrate d'alumine selon l'une quelconque des revendications 1 à 15, à la fabrication des zéolites et notamment de la zéolite A pour lessive.

**17)** Application d'hydrate d'alumine selon l'une quelconque des revendications 1 à 8 et 10 à 13, à la fabrication des marbres artificiels.

**18)** Application d'hydrate d'alumine selon l'une quelconque des revendications 1 à 8 et 10 à 15, à la fabrication des sels d'aluminium cristallisés et notamment de sulfate d'aluminium.

**19)** Application d'hydrate d'alumine selon l'une quelconque des revendications 1 à 8 et 10 à 13, comme charge ignifuge dans les matières plastiques.

**20)** Application de l'hydrate d'alumine selon l'une quelconque des revendications 1 à 8 et 10 à 13, comme charge dans les pâtes dentifrices.

Office européen des brevets

# RAPPORT DE RECHERCHE EUROPEENNE

Numero de la demande
EP 94 42 0249

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | GB-A-2 248 841 (AEI CABLES)<br>* revendications 1-3,5,6 *<br>--- | 1,2,19 | C09C1/40<br>C09C3/08<br>C09B67/00<br>A61K7/16<br>C11D3/12<br>C08K9/04<br>C04B14/30<br>C01F7/74 |
| A | EP-A-0 266 248 (RHONE-POULENC CHIMIE)<br>* page 4 - page 7; revendications 1-3 *<br>* revendications 4,5,21 *<br>--- | 1,2,4-6,<br>8,10-15 | |
| A | DE-A-23 38 759 (BAYER)<br>--- | | |
| A | US-A-5 106 420 (C. J. MARSHALL)<br>--- | | |
| A | PATENT ABSTRACTS OF JAPAN<br>vol. 10, no. 310 (C-379) 22 Octobre 1986<br>& JP-A-61 120 804 (FUKUBI KAGAKU KOGYO) 7 Juin 1986<br>* abrégé *<br>----- | 1,17 | |

| DOMAINES TECHNIQUES RECHERCHES (Int.Cl.6) |
|---|
| C09C<br>C09B |

Le présent rapport a été établi pour toutes les revendications

| Lieu de la recherche | Date d'achèvement de la recherche | Examinateur |
|---|---|---|
| LA HAYE | 2 Décembre 1994 | Van Bellingen, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)